# EUROPEAN PATENT SPECIFICATION

(11) **EP 3 801 243 B1**
(45) Date of publication and mention of the grant of the patent: **20.04.2022**
(21) Application number: 18729063.0
(22) Date of filing: 24.05.2018
(51) Int. Cl.: A61B 5/0531, A61B 5/00

(54) **IMPEDANCE MEASUREMENT DEVICE**
IMPEDANZMESSVORRICHTUNG
DISPOSITIF DE MESURE DE L'IMPÉDANCE

(43) Date of publication of application: 14.04.2021
(73) Proprietor: Scibase AB, 103 67 Stockholm (SE)
(72) Inventor: MELIN, David, 131 50 SALTSJÖ-DUVNÄS (SE); ASKARY LORD, Nima, 117 56 STOCKHOLM (SE); KORSFELDT, Angelica, 187 43 Täby (SE); JOSEFSSON, Alexandra, 112 58 STOCKHOLM (SE); BERNDTSSON, Andreas, 175 46 JÄRFÄLLA (SE)
(74) Representative: Groth & Co. KB
(86) International application number: PCT/EP2018/063706
(87) International publication number: WO 2019/223874

(56) References cited:
- US-A1- 2001 051 774
- US-A1- 2009 171 236
- US-A1- 2010 191 141
- US-A1- 2011 282 180
- US-A1- 2013 131 539

## Description

### Technical field

The present invention generally relates to the field of diagnosis of biological conditions and to a probe, medical device for non-invasively measuring impedance of tissue of living subjects and for using the measured impedance in the diagnosis of biological conditions of the tissue, for example, the presence of skin cancer, e.g. malignant melanoma or basal cell carcinoma. In particular, the present invention provides impedance data having an improved spatial resolution, both with regard to depth and lateral extension, which enables a detection of diseased skin conditions, such a malignant melanoma, at an early stage and at a high accuracy.

### Background art

Electrical impedance is a very sensitive indicator of minute changes in organic and biological material and especially tissues such as mucous membranes, skin and integuments of organs, including changes due to irritation of caused by different reactions. Therefore, significant efforts have been made to find a convenient way to measure variations and alterations in different kinds of organic and biological material to be able to establish the occurrence of such alterations that are due to different states, characteristics or irritations from e.g. diseases. Such disease includes Squamos cell carcinoma (SCC), malignant melanoma, and basal cell carcinoma (BCC), which is the most common skin cancer. Its incidence is increasing in many countries throughout the world. Exposure to ultraviolet light or ionizing radiation increases the risk for developing BCC and other tumours as well as long term immunosuppression in connection with, for example, an allogeneic organ transplantation. There seems to be no apparent genetic connection and in many patients no other predisposing factors have been found. Traditionally, skin tumours, such as malignant melanoma, have been diagnosed by means of ocular inspection by the dermatologist, in combination with skin biopsy. However, clinical diagnosis of skin tumours is proven to be difficult even for experienced dermatologists, especially in the case of pigmented lesions. In the clinic there is thus a need for a diagnostic aid besides the established method of ocular inspection by the dermatologist in combination with skin biopsies for histological examination.

In light of this, significant work has been done in order to develop diagnostic tools for the diagnosis of tumours in the skin based on impedance measurements. In WO 92/06634 a device for non-invasive measurement of electrical impedance of organic and biological material is presented. The device includes a probe having a number of concentric ring electrodes. The electrodes are driven from a control unit in such a way that the electrical current path defining the actual tissue under test is pressed towards the surface of the tissue part under test. By varying a control signal it is possible to select the region to be tested. The capability of a control electrode to vary depth penetration is limited by the shapes, sizes and distances of the electrodes and the dominating factor determining the depth penetration is distance between the electrodes.

WO 01/52731 discloses a medical electrode for sensing electric bio-potentials created within the body of a living subject. The electrode comprises a number of micro-needles adapted to penetrate the skin. The micro-needles are long enough to reach the stratum cornium and penetrate at least into the stratum corneum and are electrically conductive on their surface and connected to each other to form an array. In EP 1 437 091, an apparatus for diagnosis of biological conditions using impedance measurements of organic and biological material is disclosed. The apparatus comprises a probe including a plurality of electrodes, where each electrode is provided with a number of micro-needles each having a length being sufficient to penetrate at least into stratum corneum. The micro-needles according to EP 1 437 091 are also "nail-like", i.e. they have stem having a substantially circular cross-section with a constant or a gradually decreasing diameter and a tip-portion with a substantially spherical or needle-shaped tip.

Document US2013131539 discloses a device for quality assessment of an electrical impedance measurement on tissue.

However, clinical experience has shown that lesions, especially in early stages, include very small malignant parts, sometimes being of the magnitude down to 1 mm or less. It has further been shown that it is very difficult or almost impossible to identify such small malignant parts of diseased tissues using the prior art methods and devices due to the limited or coarse spatial resolution, both with regard to tissue depth and with regard to a lateral dimension of the tissue (i.e. in tissue layer being parallel with the surface of the skin), in the impedance spectra obtained by means of these prior art methods. It is important to detect the diseased condition, e.g. malignant melanoma, at an early stage, since the prognosis for the patient will be improved significantly since proper treatment can be initiated when the malignant part still is small. Hence, there is an evident risk using the prior art methods that diseased skin conditions such as malignant melanoma at early stage conditions are not observed due to this limited or coarse spatial resolution.

In prior art solutions reference measurements are used to further improve the accuracy in the impedance measurements, a common approach is to perform the reference measurements on a skin or tissue area close to the lesion or suspected skin or tissue spot. However, the tissue properties often vary to a high degree even between neighboring skin or tissue areas, for example due to skin properties and/or underlying structures such as bone. This entails that the data obtained in the reference measurement in fact in many cases do not deliver accurate reference information or even deliver erroneous reference information. One example is when measurements are performed in the face of a patient, where the tissue and/or skin properties vary largely between neighboring areas, for example, on the cheek from very soft to very hard depending on the cheekbone. Another example is when parts of the skin has been more exposed to weather than other neighboring parts.

In light of this, there is a need within the art of a device and method that provides improved accuracy in the obtained impedance spectra in order to enable improved detection of diseased conditions such as malignant melanoma at an early stage.

### Summary of the invention

An object of the present invention is to present an improved medical device for measuring human skin impedance with a high degree of accuracy and reliability.

Another object of the present invention is to provide an improved medical device for diagnosing diseased conditions in the human skin, such as skin cancer, with an increased accuracy and reliability.

A further object of the present invention is to provide an improved medical device for eliminating or significantly reducing the human factor or user impact in measurements of human skin impedance.

Yet another object of the present invention is to provide an improved medical device alleviating and facilitating the measurement procedures and burden both the patient and the user in measurements of human skin impedance.

These and other objects of the present invention are achieved by a device as claimed in the independent claim 1.

Further embodiments are defined in the dependent claims.

The present invention is based on a deeper understanding of dielectric properties of various tissues, which now makes it possible to ascribe a level of atypia to a spectrum of a lesion based on impedance measurements without using a nearby reference. Instead, the inventors has realized that the useful and important information can be extracted from the measured impedance spectrum on the lesion the need of a reference measurement can be eliminated. The advanced data processing with simplified measurement procedures (no reference measurements are required) improves the overall diagnostic accuracy, because the errors inherent in the reference spectrum is now eliminated. The probability in the diagnosis what a lesion really is can be improved by eliminating the errors: operator's handling errors during a number of measurements, as well as the non-ideal proxy assumption of a nearby location.

According to a first aspect of the present invention, there is provided a medical device for diagnosing a diseased condition of tissue of a subject, comprising a probe comprising a plurality of electrodes adapted to be placed in contact with the tissue, wherein each electrode comprising a plurality of micro-needles, wherein the micro-needles are adapted to penetrate the stratum corneum when the electrodes are placed against a surface of the subject. An impedance measuring circuit is configured to initiate an impedance measurement session including passing an electrical current through at least one pair of electrodes of the probe to obtain values of skin impedance of a target tissue region, the data comprising a plurality of impedance values measured in the target tissue region at different tissue layers. Further, a diagnosing unit is configured to apply a trained evaluation procedure for diagnosis of the diseased condition in the target tissue region on the basis of the measured data set of impedance values for the target tissue region, the diagnosing unit including an evaluation circuit configured to extract data related to underlying structure at the measurement site that includes data regarding a hardness or softness of the underlying structure from the measured impedance spectra, wherein the evaluation circuit is configured to determine the underlying structure to be hard if a measured impedance spectra comprises lower impedance magnitude values than normal impedance magnitude values for skin and to determine the underlying structure to be soft if a measured impedance spectra ;comprises higher impedance magnitude values than normal impedance magnitude values for skin. The trained evaluation procedure is configured to extract impedance data from the impedance spectra from obtained data sets of impedance values reflecting tissue characteristics of a lesion, determine the impedance magnitude in the obtained data sets of impedance values, evaluate the obtained data set of impedance to provide a first outcome indicating a probability of a diseased condition, and re-evaluate the first outcome based on extracted impedance data and impedance spectra data related to underlying structure at the measurement site on the skin to provide a second modified outcome indicating the probability of the diseased condition, wherein the first outcome is modified to take into account the underlying structure's impact on the measurements, wherein a first impedance magnitude compensation is performed when the extracted impedance data from the impedance spectra from obtained data sets of impedance values indicating a low impedance magnitude of the lesion and the underlying structure to be hard; and a second impedance magnitude compensation is performed when the extracted impedance data from the impedance spectra from obtained data sets of impedance values indicates a high impedance magnitude of the lesion and the underlying structure to be soft, wherein the first impedance magnitude compensation is smaller than the second level of compensation.

A main focus of the present invention is diagnosis of biological conditions of the tissue and, in particular, the presence of skin cancer, e.g. malignant melanoma or basal cell carcinoma.

However, skin barrier integrity can be evaluated/quantified using similar procedures as described above. An impaired skin barrier is a pre-cursor to many disorders such as atopic dermatitis. Thus, skin barrier changes can be detected by means of the present invention and disorders such as atopic dermatitis can be predicted at an early stage e.g. on infants, youth, and adults. Further, the efficiency of various treatments of such diseases can be assessed. Quantifying degree of sensitivity for allergens and toxic/irritant substances, both on skin and oral cavity is further applications of the present invention. Monitoring treatment of skin diseases such as psoriasis (in addition to eczema) and assessing lesions such as lichen in the oral cavity are yet other conceivable applications of the present invention. In addition, the present invention can be used to assess periodontitis to e.g. quantify risk of loss of teeth.

In embodiments of the present invention, the step of re-evaluating the first outcome based on the extracted impedance data and the data related to the underlying structure to provide a second final outcome indicating a probability of a diseased condition, comprises determining an impedance magnitude in the obtained data sets of impedance values performing a first impedance magnitude compensation when the extracted impedance data from the impedance spectra from obtained data sets of impedance values indicating a low impedance magnitude of the lesion and performing a second impedance magnitude compensation when the extracted impedance data from the impedance spectra from obtained data sets of impedance values indicates a high impedance magnitude of the lesion, wherein the first impedance magnitude compensation is smaller than the second level of compensation. The clinical data may be related to the subject, for example, the age of the subject, and the clinical data may further be based on earlier measurements of skin impedance of at least one patient or subject, or a number of measurement on a number of patients. The clinical data may stored in a database, in a cloud-based environment, in a computer based device or in a hand-held device.

In embodiments of the present invention, a pressure applying unit is adapted to apply a predetermined pressure on the tissue or skin surface of the object where the probe is placed during a measurement session and the predetermined pressure on the surface of the object where the probe is constant during the impedance measurement session.

In embodiments of the present invention, the pressure during the impedance measurements session is mechanically applied. According to the present invention, the step of revaluating the first outcome based on the extracted impedance data and the data related to underlying structure to provide a second final outcome indicating a probability of a diseased condition, comprises determining an impedance magnitude in the obtained data sets of impedance values; and performing a first impedance magnitude compensation when the extracted impedance data from the impedance spectra from obtained data sets of impedance values indicating a low impedance magnitude of the lesion; and performing a second impedance magnitude compensation when the extracted impedance data from the impedance spectra from obtained data sets of impedance values indicates a high impedance magnitude of the lesion, wherein the first impedance magnitude compensation is smaller than the second level of compensation. That is, a low impedance or magnitude indicates hard underlying structure and entails a smaller compensation compared to a case with higher impedance which, on the other hand, indicates softer underlying structure and thus a higher compensation.

According to the present invention, the tissue data includes data related to hardness and/or softness of the underlying structure at the measurement site, wherein data indicating a hard underlying structure entails the first impedance magnitude compensation and data indicating a soft underlying structure entails the second impedance magnitude compensation.

In embodiments of the present invention, the predetermined pressure on the surface of the object where the probe is constant during the impedance measurement session and, preferably, the pressure during the impedance measurements session is mechanically applied. The constant predetermined pressure may be combined with or replaced by a sucking action which thus attach the probe at the tissue or skin during the measurement.

According to embodiments of the present invention, the impedance data is pre-processed, for example, by reduction of noise content and/or reduction of the dimensionality. The noise reduction may include removal of artefacts and/or reduction of noise in the impedance magnitude and/or phase angle spectra.

A pre-filter may be configured to reject measurements that do not fulfill one or a few specific criteria, such as cut-offs. The pre-filter may be applied on impedance data that has been corrected/adjusted e.g. by pre-processing as discussed above.

A classifier may be configured to assess whether quality of measured impedance data is good. This procedure may be combined with pre-processing and/or pre-filtering to further improve quality of the data.

The probe for measuring electrical impedance of tissue of a subject according to the present invention comprises a plurality of electrodes, the electrodes being adapted to be placed in direct contact with the skin of the subject, and being connectable to an impedance measuring circuit adapted to apply a voltage and to measure a resulting current to determine an impedance signal. In preferred embodiments, the probe further comprises a switching circuit for selectively activate electrode pairs by connecting at least two of electrodes with the impedance measuring circuit and disconnecting the remaining electrodes from the impedance circuit, wherein the voltage is applied at the two electrodes and the resulting current is measured between the at least two electrodes. The switching circuit is adapted to receive control signals instructing the switching circuit to activate electrode pairs in accordance with a predetermined activation scheme, the predetermined activation scheme including to activate adjacent electrodes in a successive manner to gradually scan tissue of the subject at a first tissue depth so as to obtain a sequence of impedance signals from a selected tissue depth.

According to embodiments, the probe is provided with electrodes that have an elongated rectangular shape and are arranged at the probe in parallel rows. However, there are a number of alternative designs. For example, the electrodes may be arranged as concentric rings, or as squares. The electrodes may be arranged with micro-needles wherein each electrode comprises at least one spike. The spikes are laterally spaced apart from each other and having a length being sufficient to penetrate at least into the stratum corneum. In an alternative embodiment, the electrodes are non-invasive and each electrode has a substantially flat surface adapted to be placed against the tissue of the subject. It is also possible to combine electrodes provided with micro-needles with non-invasive electrodes.

In embodiments of the present invention, the probe may have a spherical shape, i.e. the surface of the probe provided with electrodes is spherically shaped.

As the skilled person realizes, steps of the methods disclosed herein are suitable to realize as computer program or as a computer readable medium.

Generally, all terms used in the claims and description are to be interpreted according to their ordinary meaning in the technical field, unless explicitly defined otherwise herein. All references to "a/an/the [element, device, component, unit, means, step, etc.]" are to be interpreted openly as referring to at least one instance of the element, device, component, unit, means, step, etc., unless explicitly stated otherwise. The steps of any method disclosed herein do not have to be performed in the exact order disclosed unless explicitly defined otherwise herein.

Further objects and advantages of the present invention will be discussed below by means of exemplifying embodiments.

### Brief description of the drawings

Exemplifying embodiments of the invention will be described below with reference to the accompanying drawings, in which:
Fig. 1 is a schematic block diagram of one embodiment of a medical device according to the present invention; and
Fig. 2 illustrates an embodiment of a method for diagnosis of biological conditions.

### Description of exemplifying embodiments

The following is a description of exemplifying embodiments in accordance with the present invention. This description is not to be taken in limiting sense, but is made merely for the purposes of describing the general principles of the invention. Even though particular types of probes including micro-invasive as well as non-invasive will be described, the invention is also applicable to other types of such as invasive probes.

Thus, preferred embodiments of the present invention will now be described for the purpose of exemplification with reference to the accompanying drawings, wherein like numerals indicate the same elements throughout the views. It should be understood that the present invention is defined in the appended claims.

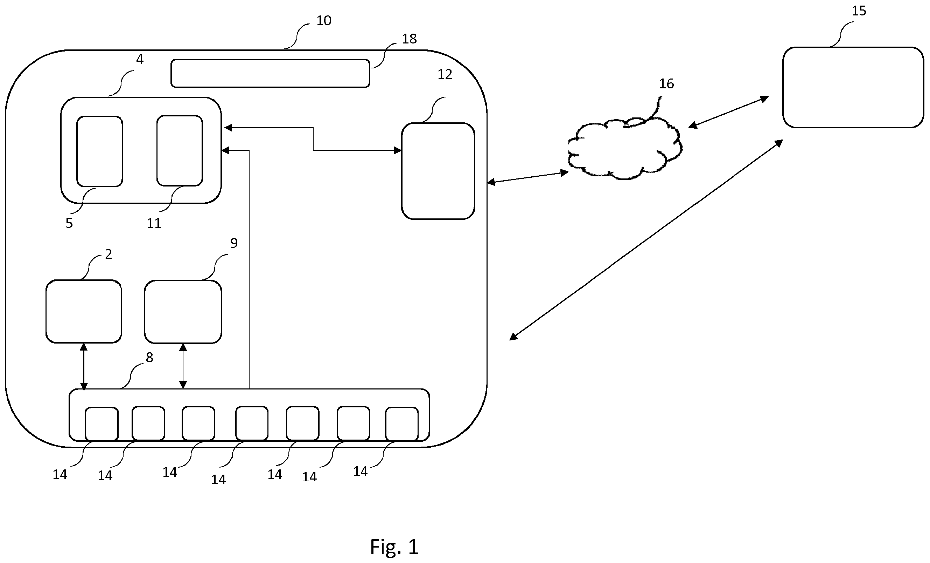

Referring first to Fig. 1, a general description a medical device according to the present invention will be discussed. The device 10 comprises an impedance measuring circuit or unit 2 and a diagnosing unit 4 for diagnosing the diseased condition in the tissue on basis of measured impedance data. The impedance measuring unit 2 is adapted to obtain impedance data of a target tissue region of the tissue of the subject. It is to be understood that the impedance data of the target tissue region comprises a plurality of impedance values obtained at different tissue depths (or layers) over a spectrum of frequencies. According to preferred embodiments of the present invention, the tissue of the subject comprises skin of the subject. However, the method and device described herein could equally well be applied to a tissue biopsy (e.g. test sample) or to a point under the skin of a subject (subcutaneously), by means of, for example, pointed electrodes. By the target tissue region it is meat a tissue region which is to be diagnosed, for example, a tissue region that is suspected of being afflicted by a diseased condition. The target tissue surface may be soaked prior to the impedance measurement using for example 0.9% saline solution. For instance, the surface may be soaked for about 30 seconds prior to the electrical impedance measurements being carried out.

The tissue impedance measurement for obtaining the impedance data of the target tissue region is performed by means of a probe 8 integrated in the medical device 10 or a probe being external to the medical device 10 and connected to the medical device 10. Irrespective of being external or integrated, the probe comprises a plurality of electrodes 14 adapted to be placed in contact with the tissue to be analyzed, typically skin of the subject. The tissue impedance may be measure by applying an AC voltage over a pair of electrodes and measure the resulting current passing over the same pair of electrodes. In embodiments of the present invention, the probe 8 comprises five electrodes, e.g. shaped as rectangular electrode bars. The electrodes are adapted to be placed in direct contact with the skin. Adjacent electrodes are separated with a distance of about 0.3 mm and having a length of about 5 mm, has shown to be a practical and useful configuration for detections of diseased conditions such as malignant melanoma, both with regard to spatial resolution in a lateral dimension and in a depth dimension. A skin area of about 5×5 mm or about 25 mm² is thus covered by the probe and at high frequencies, above about 100 kHz, the deepest tissue layer being reached is about 2.5 mm which has been proven to be a clinical relevant depth. In order to cover a larger skin area, the probe can be moved to a neighboring skin site. However, as the skilled person realizes, the probe may include more or less than five electrodes, for example 3 or 7 electrodes. Further, other electrode dimensions and other spacing between adjacent electrodes are conceivable, for example, electrodes having a width of about 4 mm and a length of about 8 mm.

By selecting adjacent pairs of electrodes, the topmost layer of the skin can be scanned in steps, and by selecting pairs that are spaced further apart, i.e. electrode pairs with one or more intermediate electrodes, the resulting current path allows for measurement at deeper skin layers. In this exemplifying embodiment of the probe according to the present invention, there are ten possible ways of selecting electrode pairs. The possibility to measure inter alia the topmost skin layer in small (determined inter alia by the spacing between adjacent electrodes and the frequency of the applied current) consecutive partitions is important since it allows for detection of small anomalies in the skin and tissue. Each electrode of the probe may be set in four different states including inject (the electrode is set to inject measurement current into the tissue), measure (the resulting current from the tissue is measured via the electrode), ground (the electrode is grounded to prevent leakage of superficial current when measurements are performed using other electrodes) and floating (the electrode is disconnected).

The diagnosing unit 4 may include storage units (not shown) for storing, for example, obtained impedance data performed on the patient. The diagnosing unit 4 may also include a processing circuit 5, in this embodiment included in the diagnosing unit 4, adapted to process obtained impedance data to reduce the number of variables by removing insignificant variables by performing linear or non-linear projections of the impedance data to lower subspaces. In preferred embodiments of the present invention, principal component analysis (PCA) is used. An alternative approach is to use parallel factor analysis (PARAFAC). Further, classification rules determined by means of, for example, linear discriminant analysis (LDA) or soft independent modelling of class analogy (SIMCA) may be used to improve the diagnosing, see for example, "Skin cancer as seen by electrical impedance", P. Åberg, Department of Laboratory Medicine, Karolinska Institutet, Stockholm, Sweden, 2004.

Moreover, the diagnosing unit 4 may communicate with display means (not shown) for displaying a diagnosis result from the diagnosis. The diagnosing unit 4 analyses the obtained and processed impedance spectrum, including impedance data obtained at different tissue depths and at different locations in relation to the probe as have been described above and at different frequencies, to provide a diagnosis of a diseased condition of the skin, for example, basal cell carcinoma, squamous cell carcinoma, or malignant melanoma.

According to embodiments of the present invention, each electrode is provided with micro-needles, thereby forming a micro-needled surface. As has been discussed above, the probe, in preferred embodiments, may include five rectangular areas or bars. In this configuration, each bar contains an array of, for example, 57 (19 × 3) micro-needles. Each bar is about 1 mm wide and 5 mm long. The distance between adjacent bars is about 0.2-0.5 mm. The active part of the probe is thus about 5 × 5 mm. Each micro-needle has a length of approximately 100 micrometer, as measured from its base, and a thickness of at least 20 micrometer. The electrode bars and micro-needles can be made of plastic material in a moulding process. The material could be made intrinsically conductive or covered with a conductive layer such as gold. In an alternative embodiment, the electrode bars and micro-needles are made of silicon and covered with gold having a thickness of at least 2 micrometer. However, other materials comprising a conductive surface with similar dimensions would work, but it should be selected to be biocompatible. In, for example, the patent applications EP 1959828, EP 1600104, and EP 1437091 by the same applicant, different probe concepts having such micro-needles are described.

In another embodiment, the electrode bars are non-invasive and substantially flat. In, for example, US 5,353,802 by the same applicant, a probe concept including non-invasive electrodes has been described.

In other embodiments of the present invention, the probe is spherically shaped, i.e. the surface including the electrodes that is pressed against the skin or tissue during a measurement has a spherical shape. This also means that the electrodes may be at least partly spherically shaped.

Human skin is a complex heterogeneous and anisotropic multilayer structure having electronically non-linear properties. Particularly the Stratum Corneum (that is the outermost layer of the epidermis), below which diseases such as skin cancer and allergic reactions manifest, is characterized by highly non-linear effects and very high electrical impedance. Thus, depending on the particular design of the measurement probe, non-invasive electrical impedance spectra of skin may be dominated by the dielectric properties of Stratum Corneum, especially at low frequencies. Furthermore, the Stratum Corneum has large and broad so called alpha dispersion that may lead to responses from underlying viable skin layers that may be confounded with responses from Stratum Corneum, that may dilute the clinically relevant information from the viable skin. For example, each spike may have a length of 0.01 to 1 mm for cancer assessments, whereas other tissues and organs, which may be encapsulated with a thicker envelope requires longer spikes or micro-needles. The spikes may be arranged on electrodes, in turn arranged on the probe, where each electrode may comprise from at least two spikes to about 100-200 spikes in certain applications, and any number in between. In the patent US 9,636,035 by the same applicant, examples of preferred embodiments of spike designs are described. By such configurations of spikes an increased versatility and increased adaptability in terms of capacity requirements can be achieved, in addition to possibly alleviating the problem of non-linear effects of Stratum Corneum.

A control circuit 9 may be configured to control, for example, switching cycles/sequences of the electrodes 14 in accordance with a predetermined activation procedure or scheme. This predetermined activation scheme may include an activation of adjacent electrode in a successive manner to gradually scan tissue of the subject at a first tissue depth, which scanned tissue depends to a large extent on spacing between activated electrode pairs so as to obtain a matrix of impedance signals from different tissue depths.

The diagnosing unit 4 is configured to pre-process the impedance data, for example, reduction of noise content and/or reduction of the dimensionality. The noise reduction may include reduction of noise in the impedance magnitude and/or phase angle spectra. The noise reduction may for example be made with the use of a Savitsky-Golay smoothing filter. Data on the subject's physical conditions may also be utilized by the diagnosing unit 4 and the data on the physical conditions may be parameterized and further used in the diagnosing process. Further, the pre-processing may comprise detection and correction of spikes or other artefacts, enabling removal of spikes or artefacts in the impedance spectrum, i.e. magnitude and/or phase angle spectra. Spikes may for example be detected with a median filter with an adequate window size. Data points of the filtered data that differ too much from raw data may be considered to be a spike or other artefact and may be corrected by e.g. linear interpolation.

The diagnosing unit 4 may further comprise a pre-filter enabling rejection of measurement that do not fulfill one or a few specific criteria, such as cut-offs. The pre-filter may be applied on impedance data that has been corrected/adjusted e.g. by pre-processing as discussed above. For example, the magnitude values and/or phase angle values may all be required to fall within a specified magnitude range of a specified phase range, respectively, in order for a measurement not to be rejected. If the measurement is on a human/animal skin, the criteria, such as the ranges, may be set such as non-physiological measurements are rejected. Also, a specific criteria may be set for a certain value relating to a specific frequency.

The diagnosing unit 4 may further include a classifier to assess whether quality of measured impedance data is good. This procedure may be combined with pre-processing and/or pre-filtering to further improve quality of the data. Examples of such classification include assessment of the variation, e.g. the variance or standard deviation, of magnitude and/or phase angle in different permutations at one or a plurality of frequencies. Another examples, include the absolute value of magnitude and/or phase angle, the median value or average value, or skewness of magnitude or phase angle.

Further, the diagnosing unit 4 include an evaluation circuit 11 configured to provide a diagnosis of a diseased condition in the target tissue region on the basis of the measured data set of impedance values for the target tissue region. According to the invention, the evaluation circuit 11 includes a trained evaluation procedure for diagnosis of the diseased condition in the target tissue region. The trained evaluation procedure performs extracting impedance data from the impedance spectra from obtained data sets of impedance values reflecting tissue characteristics of a lesion, evaluating the obtained data set of impedance to provide a first outcome indicating a probability of a diseased condition. The evaluation circuit is further configured to extract or obtain data from the impedance spectra indicating whether the underlying structure is hard or soft, which data is used in the re-evaluation of the first outcome to compensate or modify the first outcome to also take into account the underlying structure's impact on the measurements. In order to determine whether the underlying structure is hard versus soft the circuit may be configured to determine the underlying structure to be soft if a measured impedance spectra comprises higher impedance magnitude values than normal impedance magnitude values for skin. In the corresponding manner, the circuit may be configured to determine the underlying structure to be hard if a measured impedance spectra comprises lower impedance magnitude values than normal impedance magnitude values for skin. A low impedance or magnitude thus indicates hard underlying structure and entails a smaller compensation compared to a case with higher impedance which, on the other hand, indicates softer underlying structure and thus a higher compensation.

Further, the trained evaluation procedure re-evaluates the first outcome based on the extracted impedance data and data related to the measurement site, e.g. whether the underlying structure is hard or soft, and, in some embodiments, clinical data such as age of subject, to provide a second outcome indicating a final or compensated probability of a diseased condition. The data related to hardness/softness of the underlying structure is extracted from the measured impedance spectra (for example, by comparison with stored data values from a number of patients). The re-evaluation of the first outcome based on the extracted impedance data and the data related to the underlying structure to provide a second final outcome indicating a probability of a diseased condition may comprise determining an impedance magnitude in the obtained data sets of impedance values and performing an impedance magnitude compensation based on the determined impedance magnitude.

In embodiments of the present invention, the re-evaluation of the first outcome based on the extracted impedance data and the data related to underlying structure to provide a second final outcome indicating a probability of a diseased condition comprises determining an impedance magnitude in the obtained data sets of impedance values, performing a first impedance magnitude compensation when the extracted impedance data from the impedance spectra from obtained data sets of impedance values indicating a low impedance magnitude of the lesion, and performing a second impedance magnitude compensation when the extracted impedance data from the impedance spectra from obtained data sets of impedance values indicates a high impedance magnitude of the lesion, wherein the first impedance magnitude compensation is smaller than the second level of compensation.

In embodiments of the present invention, the clinical data may be based on earlier measurements of skin impedance of at least one patient, or preferably on aggregated information from a number of measurement and patients. This clinical data may be stored in a database, in a cloud-based environment, in a computer based device or in a hand-held device.

The medical device 10 further include a communication unit 12 capable of transmitting/receiving data to/from external units 14, such as a laptop computer, a handheld computer/device, a database, a cloud-based arrangement, etc., directly with the unit or network itself or via a wireless network 16. In this way, the device 10 may be supplied with, for example, clinical data for use in the diagnosis such as clinical data on the subject's physical condition, that may include, but is not limited to, subject's age, lesion ABCDE characteristics, gender, lesion size, location of the lesion etc. Moreover, data obtained with the medical device 10 such as impedance data from measurements can also be downloaded to external devices 14 via the communication unit 12.

Furthermore, the medical device 10 includes a pressure applying unit 18 configured to apply a predetermined pressure on the tissue or skin when activated and the probe 8 is pressed against the tissue or skin during the measurement. Preferably, the pressure is constant during the measurement session. For example, a pressure in a range of 2 - 12 N may be applied, or in preferred embodiments a pressure in a range of 3 - 10 N, or in further preferred embodiments in a range of 5 - 7 N or as in a certain embodiments in a range of 5.5 - 6.5 N. In embodiments of the present invention, the applied predetermined pressure may be combined with or replaced by a sucking action, which thus attached the probe to the tissue or skin during the measurement.

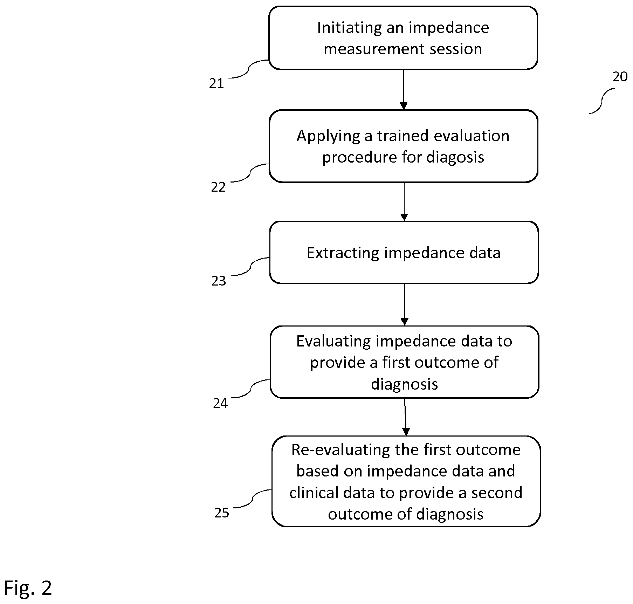

With reference now to Fig. 2, an embodiment of a method will be described. The method 20 for diagnosing a diseased condition of tissue of a subject uses a medical device, for example, a device as described above with reference to Fig. 1 includes initiating 21 an impedance measurement session including passing an electrical current through the electrodes to obtain values of skin impedance of a target tissue region, which data comprises a plurality of impedance values measured in the target tissue region at different tissue layers. In this embodiment, a predetermined pressure can be applied on the tissue or skin surface of the object where the probe is placed during the measurement session. Thereafter, a trained evaluation procedure is applied 24 for diagnosis of the diseased condition in the target tissue region on the basis of the measured data set of impedance values for the target tissue region. Further, impedance data from the impedance spectra from obtained data sets of impedance values reflecting tissue characteristics of a lesion is extracted 23. The obtained data set of impedance is evaluated 24 to provide a first outcome indicating a probability of a diseased condition. Data related to underlying structure, e.g. hardness/softness, is obtained, calculated or extracted from the impedance spectra, for example. Based on this, the first outcome is reevaluated 25 to provide a second final outcome indicating a probability of a diseased condition. However, the data related to the underlying structure can be obtain/extracted in connection with the measurements or when determining a first outcome or a final outcome, or in between any of these steps.

It is to be understood that in the context of the present invention and in relation to electrical components electrically connected to each other, the term connected is not limited to mean directly connected, but also encompasses functional connections having intermediate components. For example, on one hand, if an output of a first component is connected to an input of a second component, this comprises a direct connection. On the other hand, if an electrical conductor directly supplies a signal from the output of the first component substantially unchanged to the input of the second component, alternatively via one or more additional components, the first and second components are also connected. However, the connection is functional in the sense that a gradual or sudden change in the signal from the output of the first component results in a corresponding or modified change in the signal that is input to the second component.

Although exemplary embodiments of the present invention has been shown and described, it will be apparent to those having ordinary skill in the art that a number of changes, modifications, or alterations to the inventions as described herein may be made. Thus, it is to be understood that the above description of the invention and the accompanying drawings is to be regarded as a non-limiting example thereof and that the scope of protection is defined by the appended patent claims.

## Claims

1. A medical device (10) for diagnosing a diseased condition of tissue of a subject, comprising:
a probe (8) comprising a plurality of electrodes (14) adapted to be placed in contact with the tissue, wherein each electrode (14) comprising a plurality of micro-needles, wherein said micro-needles are adapted to penetrate the stratum corneum when said electrodes are placed against a surface of the subject;
an impedance measuring circuit (2) configured to initiate an impedance measurement session including passing an electrical current through at least one pair of electrodes of said probe (8) to obtain values of skin impedance of a target tissue region, said data comprising a plurality of impedance values measured in the target tissue region at different tissue layers;
a diagnosing unit (4) configured to apply a trained evaluation procedure for diagnosis of said diseased condition in the target tissue region on the basis of the measured data set of impedance values for the target tissue region, said diagnosing unit (4) including an evaluation circuit (11) configured to extract data related to underlying structure at the measurement site that includes data regarding a hardness or softness of the underlying structure from the measured impedance spectra, wherein said evaluation circuit (11) is configured to determine the underlying structure to be hard if a measured impedance spectra comprises lower impedance magnitude values than normal impedance magnitude values for skin and to determine the underlying structure to be soft if a measured impedance spectra comprises higher impedance magnitude values than normal impedance magnitude values for skin;
wherein said trained evaluation procedure is configured to:
extract impedance data from the impedance spectra from obtained data sets of impedance values reflecting tissue characteristics of a lesion;
determine the impedance magnitude in the obtained data sets of impedance values;
evaluate the obtained data set of impedance to provide a first outcome indicating a probability of a diseased condition; and
re-evaluate the first outcome based on extracted impedance data and impedance spectra data related to underlying structure at the measurement site on the skin to provide a second modified outcome indicating the probability of the diseased condition, wherein the first outcome is modified to take into account the underlying structure's impact on the measurements, wherein
a first impedance magnitude compensation is performed when the extracted impedance data from the impedance spectra from obtained data sets of impedance values indicating a low impedance magnitude of the lesion and the underlying structure to be hard; and
a second impedance magnitude compensation is performed when the extracted impedance data from the impedance spectra from obtained data sets of impedance values indicates a high impedance magnitude of the lesion and the underlying structure to be soft, wherein the first impedance magnitude compensation is smaller than the second level of compensation.

2. The device according to claim 1, further comprising a communication unit (12) capable of transmitting/receiving data to/from external units (14) directly with the communication unit (12) or via a wireless network 16, wherein clinical data comprising information of the subject and/or tissue data is obtained.

3. The device according to claim 2, wherein the clinical data comprises information based on earlier measurements of skin impedance of at least one patient or subject.

4. The device according to claim 2 or 3, wherein said clinical data is stored in a database (15), in a cloud-based environment (15), in a computer-based device (15) or in a hand-held device (15).

5. The device according to claim 1 - 4, further comprising a pressure applying unit (18) adapted to apply a predetermined pressure on the tissue or skin surface of the object where the probe (8) is placed.

6. The device according to claims 5, wherein the pressure applying unit (18) is adapted to apply a predetermined pressure on the surface of the object wherein the pressure is constant during the impedance measurement session.

## Patentansprüche

1. Medizinische Vorrichtung (10) zum Diagnostizieren eines Erkrankungszustands von Gewebe eines Subjekts, umfassend:
eine Sonde (8), die eine Vielzahl von Elektroden (14) umfasst, die eingerichtet sind, um in Kontakt mit dem Gewebe platziert zu werden, wobei jede Elektrode (14) eine Vielzahl von Mikronadeln umfasst, wobei die Mikronadeln eingerichtet sind, um das Stratum corneum zu durchdringen, wenn die Elektroden auf einer Oberfläche des Subjekts platziert sind;
eine Impedanzmessschaltung (2), die ausgestaltet ist, um eine Impedanzmesssitzung zu initiieren, die Leiten eines elektrischen Stroms durch mindestens ein Paar von Elektroden der Sonde (8) einschließt, um Werte der Hautimpedanz einer Zielgeweberegion zu erhalten, wobei die Daten eine Vielzahl von Impedanzwerten umfassen, die in der Zielgeweberegion in unterschiedlichen Gewebeschichten gemessen werden;
eine Diagnostikeinheit (4), die ausgestaltet ist, um ein trainiertes Evaluierungsverfahren zur Diagnostik des Erkrankungszustand in der Zielgeweberegion basierend auf dem gemessenen Datensatz der Impedanzwerte für die Zielgeweberegion anzuwenden, wobei die Diagnostikeinheit (4) eine Evaluierungsschaltung (11) einschließt, die ausgestaltet ist, um Daten, die mit einer darunter befindlichen Struktur an dem Messsitus zusammenhängen, die Daten einschließen, die eine Härte oder Weichheit der darunter befindlichen Struktur betreffen, aus den gemessenen Impedanzspektren zu extrahieren, wobei die Evaluierungsschaltung (11) ausgestaltet ist, um zu ermitteln, dass die darunter befindliche Struktur hart ist, wenn ein gemessenes Impedanzspektrum niedrigere Impedanzgrößenwerte als normale Impedanzgrößenwerte für Haut umfasst, und um zu ermitteln, dass die darunter befindliche Struktur weich ist, wenn ein gemessenes Impedanzspektrum höhere Impedanzgrößenwerte als normale Impedanzgrößenwerte für Haut umfasst;
wobei das trainierte Evaluierungsverfahren ausgestaltet ist zum:
Extrahieren von Impedanzdaten aus den Impedanzspektren aus erhaltenen Datensätzen von Impedanzwerten, die Gewebecharakteristika einer Läsion widerspiegeln;
Ermitteln der Impedanzgröße in den erhaltenen Datensätzen der Impedanzwerte;
Evaluieren des erhaltenen Impedanzdatensatzes, um ein erstes Ergebnis bereitzustellen, das eine Wahrscheinlichkeit eines Erkrankungszustands angibt; und
erneutes Evaluieren des ersten Ergebnisses basierend auf extrahierten Impedanzdaten und Impedanzspektrumdaten, die mit darunter befindlicher Struktur an dem Messsitus auf der Haut zusammenhängen, um ein zweites modifiziertes Ergebnis bereitzustellen, das die Wahrscheinlichkeit des Erkrankungszustands angibt, wobei das erste Ergebnis modifiziert wird, um den Einfluss der darunter befindlichen Struktur auf die Messungen zu berücksichtigen, wobei
eine erste Impedanzgrößenkompensation durchgeführt wird, wenn die extrahierten Impedanzdaten aus den Impedanzspektren aus erhaltenen Datensätzen der Impedanzwerte eine niedrige Impedanzgröße der Läsion angeben, und dass die darunter befindliche Struktur hart ist; und
eine zweite Impedanzgrößenkompensation durchgeführt wird, wenn die extrahierten Impedanzdaten aus den Impedanzspektren aus erhaltenen Datensätzen der Impedanzwerte eine hohe Impedanzgröße der Läsion angeben, und dass die darunter befindliche Struktur weich ist, wobei die erste Impedanzgrößenkompensation kleiner als das zweite Kompensationsniveau ist.

2. Vorrichtung nach Anspruch 1, des Weiteren umfassend eine Kommunikationseinheit (12), die in der Lage ist, direkt mit der Kommunikationseinheit (12) oder über ein drahtloses Netzwerk 16 Daten zu externen Einheiten (14) zu senden/von diesen zu empfangen, wobei klinische Daten, die Informationen des Subjekts umfassen, und/oder Gewebedaten erhalten werden.

3. Vorrichtung nach Anspruch 2, wobei die klinischen Daten Informationen umfassen, die auf früheren Messungen der Hautimpedanz von mindestens einem Patienten oder Subjekt basieren.

4. Vorrichtung nach Anspruch 2 oder 3, wobei die klinischen Daten in einer Datenbank (15), in einer Cloud-basierten Umgebung (15), in einer Computerbasierten Vorrichtung (15) oder in einer handgeführten Vorrichtung (15) gespeichert sind.

5. Vorrichtung nach Anspruch 1 bis 4, des Weiteren umfassend eine Druckausübungseinheit (18), die eingerichtet ist, um einen vorbestimmten Druck auf das Gewebe oder die Hautoberfläche des Objekts auszuüben, wo die Sonde (8) platziert wird.

6. Vorrichtung nach Anspruch 5, wobei die Druckausübungseinheit (18) eingerichtet ist, um einen vorbestimmten Druck auf die Oberfläche des Objekts auszuüben, wobei der Druck während der Impedanzmesssitzung konstant ist.

## Revendications

1. Dispositif médical (10) pour diagnostiquer un état pathologique d'un tissu d'un sujet, comprenant :
une sonde (8) comprenant une pluralité d'électrodes (14) conçues pour être placées en contact avec le tissu, chaque électrode (14) comprenant une pluralité de micro-aiguilles, lesdites micro-aiguilles étant conçues pour pénétrer dans la couche cornée lorsque lesdites électrodes sont placées contre une surface du sujet ;
un circuit de mesure d'impédance (2) configuré pour initier une session de mesure d'impédance comprenant le passage d'un courant électrique à travers au moins une paire d'électrodes de ladite sonde (8) pour obtenir des valeurs d'impédance de peau d'une région de tissu cible, lesdites données comprenant une pluralité de valeurs d'impédance mesurées dans la région de tissu cible au niveau de différentes couches de tissu ;
une unité de diagnostic (4) configurée pour appliquer une procédure d'évaluation formée pour le diagnostic dudit état pathologique dans la région de tissu cible sur la base de l'ensemble de données mesurées des valeurs d'impédance pour la région de tissu cible, ladite unité de diagnostic (4) comprenant un circuit d'évaluation (11) configuré pour extraire des données liées à la structure sous-jacente au niveau du site de mesure qui comprennent des données concernant une dureté ou une souplesse de la structure sous-jacente à partir des spectres d'impédance mesurés, ledit circuit d'évaluation (11) étant configuré pour déterminer que la structure sous-jacente est dure si un spectre d'impédance mesuré comprend des valeurs d'amplitude d'impédance inférieures à des valeurs d'amplitude d'impédance normales pour la peau et pour déterminer que la structure sous-jacente est souple si un spectre d'impédance mesuré comprend des valeurs d'amplitude d'impédance supérieures à des valeurs d'amplitude d'impédance normales pour la peau ;
ladite procédure d'évaluation entraînée étant configurée pour :
extraire des données d'impédance des spectres d'impédance à partir d'ensembles de données obtenus de valeurs d'impédance reflétant des caractéristiques tissulaires d'une lésion ;
déterminer l'amplitude d'impédance dans les ensembles de données obtenus de valeurs d'impédance ;
évaluer l'ensemble des données d'impédance obtenues pour fournir un premier résultat indiquant une probabilité d'un état pathologique ; et
réévaluer le premier résultat sur la base des données d'impédance extraites et des données de spectres d'impédance liées à la structure sous-jacente au niveau du site de mesure sur la peau pour fournir un second résultat modifié indiquant la probabilité de l'état pathologique, le premier résultat étant modifié pour prendre en compte l'impact des structures sous-jacentes sur les mesures,
une première compensation d'amplitude d'impédance étant réalisée lorsque les données d'impédance extraites des spectres d'impédance à partir des ensembles de données obtenus de valeurs d'impédance indiquent une faible amplitude d'impédance de la lésion et que la structure sous-jacente est dure ; et
une seconde compensation d'amplitude d'impédance étant réalisée lorsque les données d'impédance extraites du spectre d'impédance à partir des ensembles de données obtenus de valeurs d'impédance indiquent une amplitude d'impédance élevée de la lésion et que la structure sous-jacente est souple, la première compensation d'amplitude d'impédance étant inférieure au second niveau de compensation.

2. Dispositif selon la revendication 1, comprenant en outre une unité de communication (12) capable de transmettre/recevoir des données vers/depuis des unités externes (14) directement avec l'unité de communication (12) ou par l'intermédiaire d'un réseau sans fil 16, des données cliniques comprenant des informations sur le sujet et/ou des données sur les tissus étant obtenues.

3. Dispositif selon la revendication 2, les données cliniques comprenant des informations basées sur des mesures antérieures de l'impédance de peau d'au moins un patient ou sujet.

4. Dispositif selon la revendication 2 ou 3, lesdites données cliniques étant stockées dans une base de données (15), dans un environnement basé sur le cloud (15), dans un dispositif informatique (15) ou dans un dispositif portatif (15).

5. Dispositif selon la revendication 1 à 4, comprenant en outre une unité d'application de pression (18) conçue pour appliquer une pression prédéterminée sur le tissu ou la surface de la peau de l'objet où la sonde (8) est placée.

6. Dispositif selon la revendication 5, l'unité d'application de pression (18) étant conçue pour appliquer une pression prédéterminée sur la surface de l'objet, la pression étant constante pendant la session de mesure d'impédance.
